# EUROPEAN PATENT APPLICATION

(11) **EP 2 412 370 A1**
(43) Date of publication of application: **01.02.2012**
(21) Application number: 10756232.4
(22) Date of filing: 26.03.2010
(51) Int. Cl.: A61K 31/40, A61K 31/403, A61K 31/4196, A61K 47/02, A61K 47/10, A61K 47/26, A61P 43/00

(54) **MATRIX-TYPE SUSTAINED RELEASE PREPARATION CONTAINING BASIC ADDITIVE**

(30) Priority: 27.03.2009 JP 2009078087
(71) Applicant: Kyorin Pharmaceutical Co., Ltd., Tokyo 101-8311 (JP)
(72) Inventor: FUKUDA Mamoru, Chiyoda-ku Tokyo 1018311 (JP); TAKAHASHI Mayuko, Shimotsuga-gun Tochigi 329-0114 (JP); GOTO Takahiro, Shimotsuga-gun Tochigi 329-0114 (JP)
(74) Representative: Vossius & Partner
(86) International application number: PCT/JP2010/055409
(87) International publication number: WO 2010/110436

(57) **Abstract**

Disclosed is a matrix-type sustained release preparation containing a basic drug unstable against an acid as an active pharmaceutical ingredient, wherein the decomposition of the basic drug unstable against the acid can be prevented and therefore the pH-dependent release can be reduced. A hardly water-soluble basic normal salt is added to a matrix-type sustained release preparation containing a basic drug that is unstable against an acid.

## Description

### Technical Field

The present invention relates to a matrix-type sustained release preparation containing a basic drug which is unstable against an acid. More particularly, the present invention relates to a matrix-type sustained release preparation which can control the release of a basic drug irrespective of pH environment and which can inhibit decomposition of the basic drug unstable against an acid.

### Technical Background:

The development of a sustained release preparation is quite profitable for the reduction in number of administration and for the suppression of occurrence of side effects. As the sustained release preparations, there have been known, for instance, the matrix-type sustained release preparation which can gradually release the drug within the gastrointestinal tract, while the preparation is still maintained in its original dosage form; and the multiple unit-type sustained release preparation in which tablets or capsules administered are disintegrated in the body and the released granules show the desired sustained release characteristics. The present invention relates to one of the matrix-type sustained release preparations. The matrix-type sustained release preparation has such merits that the preparation thereof is easy as compared with the multiple unit-type one and that it is likewise cheaper than the latter.

Most basic drugs show higher solubility in an environment having a lower pH value (acidic side) and their release rates vary depending on the pH. Accordingly, in case of the preparation containing a basic drug, various factors frequently makes the control of the release thereof irregular depending on factors including variation in pH at various sites of the gastrointestinal tract (such as the stomach and intestine), individual difference in pH value of the digestive fluids and the variation in pH at the gastrointestinal tract caused by the foods and drugs. The release rate of a drug is reduced in an environment having a high pH value, which makes bioavailability insufficient. In particular, when it is required for a sustained release preparation to maintain the release rate of its active pharmaceutical ingredient at a constant level over a long period of time, there has been desired for the development of such a sustained release preparation whose release rate is independent of pH.
As an attempt to develop a basic drug-containing matrix-type sustained release preparation showing a pH-independent release profile, there have been known those listed below; there have been known a method in which an enteric polymer or an acidic additive is incorporated into a pharmaceutical composition as a pH-adjusting agent (Patent Documents 1, and 3 to 6 as well as Non-Patent Document 1); a method in which a pH-dependent polymer in the form of an alginate is incorporated into a pharmaceutical composition (Patent Document 2); and a method in which a basic substance is added to a pharmaceutical composition.
On the other hand, there has also been known an N-acylpyrrolidine carbonitrile derivative having an amino group as a basic DPP-IV inhibitor (Patent Documents 7 to 12). It is known that these compounds are quite susceptible to moisture and acids and that the decomposition thereof proceed due to hydrolysis (see Patent Documents 13 to 17 specified below).
However, these prior art documents do not disclose a matrix-type sustained release preparation containing an active pharmaceutical ingredient which is susceptible to moisture and acids and to the decomposition through hydrolysis and these prior art documents do not disclose a matrix-type sustained release preparation which can inhibit the decomposition of the active pharmaceutical ingredient and which has reduced pH-dependent release.

### Disclosure of Prior Literature:

### Patent Document:

Patent Document 1: WO 2006/070781 Pamphlet
Patent Document 2: JP-A-01-100134
Patent Document 3: JP-A-63-227519
Patent Document 4: JP-A-2004-518676
Patent Document 5: JP-A-06-199657
Patent Document 6: JP-A-02-223533
Patent Document 7: WO 2005/075421 Pamphlet
Patent Document 8: WO 98/19998 Pamphlet
Patent Document 9: WO 00/34241 Pamphlet
Patent Document 10: WO 2001/068603 Pamphlet
Patent Document 11: WO 2006/040625 Pamphlet
Patent Document 12: WO 2002/014271 Pamphlet
Patent Document 13: JP-A-2008-543773
Patent Document 14: JP-A-2008-527004
Patent Document 15: JP-A-2008-510764
Patent Document 16: JP-A-2007-518760
Patent Document 17: JP-A-2008-501025

### Non-Patent Document:

Non-Patent Document 1: Journal of Pharmaceutical Sciences, vol.95, No.7, July 2006, 1459-1468
Non-Patent Document 2: Journal of Pharmaceutical Sciences, vol.88, Not.11, November 1999, 1140-1148

### Disclosure of the Invention:

### Problems That the Invention is to Solve:

It is an object of the present invention to provide, as a pharmaceutical composition containing a basic drug unstable against an acid, a matrix-type sustained release preparation which can suppress any decomposition of such a basic drug unstable against acids and which can reduce pH-dependent release.

### Means for the Solution of the Problems:

The inventors of this invention have conducted intensive studies to solve the foregoing problems and as a result, have found that if a basic normal salt hardly soluble in water is incorporated into a matrix-type sustained release preparation which comprises a basic compound unstable against acids such as an N-acylpyrrolidine carbonitrile derivative having an amino group in a molecule, one can obtain a matrix-type sustained release preparation which can suppress any decomposition of the active pharmaceutical ingredient and which can reduce or improve the pH-dependent release characteristics thereof, and have thus completed the present invention.

More specifically, the present invention relates to a matrix-type sustained release preparation as will be detailed below.
[1] A matrix-type sustained release preparation wherein an N-acylpyrrolidine carbonitrile derivative having an amino group in a molecule is stabilized, said preparation comprising a basic normal salt hardly soluble in water and an excipient.

[2] The matrix-type sustained release preparation as set forth in the foregoing item [1], wherein the N-acylpyrrolidine carbonitrile derivative having an amino group in a molecule is vildagliptin, saxagliptin, melogliptin, denagliptin, TS-021, MP-513, or an aminoacetylpyrrolidine carboxamide derivative represented by the following general formula (1):

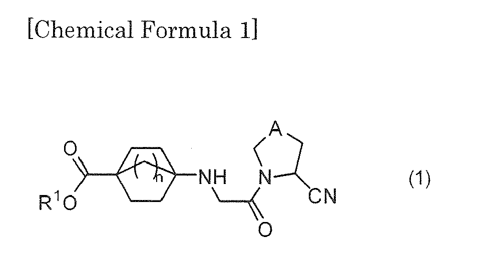

(In the general formula (1),
A represents CH₂, CHF or CF₂;
R¹ represents a hydrogen atom, a C1 to C6 alkyl group which may be substituted, a C3 to C8 cycloalkyl group which may be substituted, an arylmethyl group which may be substituted, an arylethyl group which may be substituted, an aromatic hydrocarbon group which may be substituted, an aromatic hetero ring which may be substituted, or an aliphatic hetero ring which may be substituted; and
n is 1 or 2).

[3] The matrix-type sustained release preparation as set forth in the foregoing item [1], wherein the N-acylpyrrolidine carbonitrile derivative having an amino group in a molecule is vildagliptin, saxagliptin, melogliptin, denagliptin, TS-021, MP-513, or (2S,4S)-1-[N-(4-ethoxycarbonylbicyclo[2.2.2]-oct-1-yl)amino]acetyl-4-fluoropyrrolidine-2-carbonitrile.

[4] The matrix-type sustained release preparation as set forth in any one of the foregoing items [1] to [3], wherein the basic normal salt hardly soluble in water is calcium carbonate.

[5] The matrix-type sustained release preparation as set forth in any one of the foregoing items [1] to [4], wherein the excipient is lactose or a sugar alcohol.

[6] The matrix-type sustained release preparation as set forth in any one of the foregoing items [1] to [5], wherein the excipient is lactose or mannitol.

[7] The matrix-type sustained release preparation as set forth in any one of the foregoing items [1] to [6], further comprising a pH-independent hydrogeling agent.

[8] The matrix-type sustained release preparation as set forth in any one of the foregoing items [1] to [7], wherein the amount of the hydrogeling agent to be incorporated is not more than 3 times that of the basic normal salt hardly soluble in water.

[9] The matrix-type sustained release preparation as set forth in the foregoing item [8], wherein the amount of the hydrogeling agent to be incorporated is not more than 2 times that of the basic normal salt hardly soluble in water.

### Effects of the Invention:

According to the present invention, provided is a matrix-type sustained release preparation containing, as an active pharmaceutical ingredient a basic drug unstable against acids in which the decomposition of the active pharmaceutical ingredient present is suppressed and whose pH-dependent release is reduced. The matrix-type sustained release preparation according to the present invention has gel strength on the order of the practically acceptable level.

### Mode for Carrying out the Invention:

The term "pH-dependent release is reduced" used in this specification means that the difference in the drug release rate between the stomach and the intestine is reduced. More specifically, when carrying out the dissolution test as defined in the 15^{th} revised edition of Japanese Pharmacopoeia (called Puddle Method), it is preferred that the difference between the drug release rates observed for the first and second liquids is not more than 16% and much preferably not more than 13%, over the predetermined period of time ranging from 0 to 2 hours. In this connection, a tablet orally administered in general passes through the stomach within the term ranging from 0 to 2 hours. In addition, most basic drugs in general dissolved at a higher rate in a lower pH environment and therefore, it is important to control the dissolution rate of the tablet while it still remains in the stomach. In other words, regarding the matrix-type sustained release preparation, it is considered to be important that the pH-dependent release of the sustained release preparation should be reduced in the initial stage (during 0 to 2 hours).

The term "gel strength on the order of the practically acceptable level" used in this specification means that a solid preparation shows such strength at which the tablet does not disintegrate due to the autonomic movement of the stomach and the intestine.

In this connection, this "gel strength" can be calculated according to the method disclosed in HARM TECH JAPAN Vol.20, No.13 (2004), pp. 135-140. In other words, this method comprises the steps of immersing and allowing, to stand, a tablet in 100mL of a first liquid (the first liquid specified in the 15^{th} revised edition of Japanese Pharmacopoeia, pH 1.2) or the second liquid (the second liquid specified in the 15^{th} revised edition of Japanese Pharmacopoeia, pH 6.8), while maintaining the temperature thereof at 40°C in a thermostatic chamber for a time period of 4, 8 or 16 hours; determining the diameter of the crushed tablet using a parallel plate viscometer (spreadmeter); and then dividing the diameter observed for the tablet prior to the immersion by that observed for the crushed tablet to thus calculate the gel strength of the tablet.

In order to prevent any breakage of a tablet orally administered within the gastrointestinal tract and to achieve the desired sustained release effect, the gel strength thereof, observed when it is immersed in the first liquid, is preferably not less than 0.05 and much preferably not less than 0.1 for the immersion time of 4 hours. On the other hand, the gel strength of such a tablet, observed when it is immersed in the second liquid, is preferably not less than 0.05 and much preferably not less than 0.1 for the immersion time of 8 hours.

The "amino group" appearing in the term "N-acylpyrrolidine carbonitrile derivative having an amino group in a molecule" used in this specification means a primary amino group, a secondary amino group or a C4 to C9 cyclic amino group, with a secondary amino group being more preferred.

In this connection, the term "secondary amino group" means an aliphatic or aromatic amino group in which one hydrogen atom present on the nitrogen atom is substituted and more specifically, the term means, for instance, an amino group which is substituted with a C1 to C6 alkyl group such as a methylamino group or a butylamino group; an amino group which is substituted with a C3 to C10 cyclic alkyl group such as a cyclohexylamino group, a cyclopentylamino group, an adamantylamino group or a bicyclo[2.2.2]octanylamino group; or an aromatic amino group (such as an anilyl group or a pyridylamino group).

In addition, the term "C4 to C9 cyclic amino group" herein used means a cyclic amino group which has one or more nitrogen atoms within the ring and which may optionally comprise oxygen or sulfur atoms within the ring and specific examples thereof include a pyrrolidyl group, a piperidyl group, a morpholyl group, an oxazolyl group, an azabicycloheptyl group or an azabicyclooctyl group.

Examples of "N-acylpyrrolidine carbonitrile derivatives" used in this specification are N-aminoacetylpyrrolidine carboxamide derivatives represented by the following general formula (1):

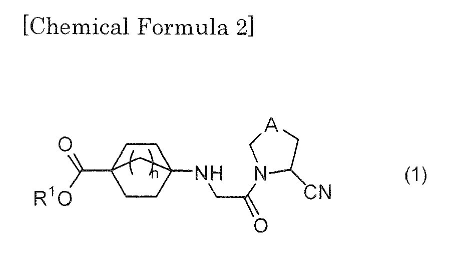

(In the general formula (1),
A represents CH₂, CHF or CF₂;
R¹ represents a hydrogen atom, a C1 to C6 alkyl group which may be substituted, a C3 to C8 cycloalkyl group which may be substituted, an arylmethyl group which may be substituted, an arylethyl group which may be substituted, an aromatic hydrocarbon group which may be substituted, an aromatic hetero ring which may be substituted, or an aliphatic hetero ring which may be substituted; and
n is 1 or 2).

The term "C1 to C6 alkyl group which may be substituted" used in this specification means a C1 to C6 alkyl group which may have 1 to 5 substituents selected from the group consisting of halogen atoms, a hydroxyl group, a cyano group, C1 to C6 alkoxy groups, aryloxy groups which may be substituted, C1 to C6 alkyl carbonyl groups, C1 to C6 alkoxy carbonyl groups, C1 to C6 alkylthio groups, amino groups, mono- or di-substituted C1 to C6 alkylamino groups, C4 to C9 cyclic amino groups each of which may have 1 to 3 hetero atoms, formylamino groups, C1 to C6 alkyl carbonylamino groups, C1 to C6 alkoxy carbonylamino groups, C1 to C6 alkylsulfonylamino groups and arylsulfonylamino groups which may be substituted.

The term "C1 to C6 alkyl group" herein used means, for instance, a linear or branched lower alkyl group such as a methyl group, an ethyl group, a propyl group, a 1-methylethyl group, a 1-methylpropyl group, a 2-methylpropyl group, a 1-ethylpropyl group, a 2-ethylpropyl group, a butyl group or a hexyl group, with an ethyl group being much preferably used herein.

The term "C3 to C8 cycloalkyl group which may be substituted" herein used means, for instance, a C3 to C8 cycloalkyl group which may have 1 to 5 substituents selected from the group consisting of halogen atoms, a hydroxyl group, a cyano group, C1 to C6 alkoxy groups, aryloxy groups which may be substituted, C1 to C6 alkyl carbonyl groups, C1 to C6 alkoxy carbonyl groups, C1 to C6 alkylthio groups, amino groups, mono- or di-substituted C1 to C6 alkylamino groups, C4 to C9 cyclic amino groups which may have 1 to 3 hetero atoms, formylamino groups, C1 to C6 alkyl carbonyl-amino groups, C1 to C6 alkoxy carbonylamino groups, C1 to C6 alkylsulfonylamino groups and arylsulfonylamino groups which may be substituted.

The term "C3 to C8 cycloalkyl group" herein used means, for instance, an alkyl group having a cycloalkyl ring such as a cyclopropyl group, a cyclopropyl methyl group, a cyclobutyl group, a cyclopentyl group, a cyclohexyl group, a cycloheptyl group, a cyclooctyl group and the like.

The term "arylmethyl group which may be substituted" herein used means an arylmethyl group (there may be mentioned, for instance, a phenylmethyl group, a naphthylmethyl group, a pyridylmethyl group, a quinolylmethyl group, an indolylmethyl group and the like), which may have 1 to 5 substituents selected from the group consisting of halogen atoms, C1 to C6 alkyl groups which may be substituted, a hydroxyl group, a cyano group, a nitro group, C1 to C6 alkoxy groups which may be substituted, aryloxy groups which may be substituted, C1 to C6 alkyl carbonyl groups, C1 to C6 alkoxy carbonyl groups, C1 to C6 alkylthio groups, amino groups, C1 to C6 alkylamino groups which may be mono- or di-substituted, arylamino groups which may be substituted, C4 to C9 cyclic amino groups which may have 1 to 3 hetero atoms, formylamino groups, C1 to C6 alkyl carbonylamino groups, C1 to C6 alkoxy carbonylamino groups, C1 to C6 alkylsulfonylamino groups and arylsulfonylamino groups which may be substituted.

The term "arylethyl group which may be substituted" used in this specification means an arylethyl group (there may be mentioned, for instance, a phenylethyl group, a naphthylethyl group, a pyridylethyl group, a quinolylethyl group, an indolylethyl group and the like), which may have 1 to 5 substituents selected from the group consisting of halogen atoms, C1 to C6 alkyl groups which may be substituted, a hydroxyl group, a cyano group, a nitro group, C1 to C6 alkoxy groups which may be substituted, aryloxy groups which may be substituted, C1 to C6 alkyl carbonyl groups, C1 to C6 alkoxy carbonyl groups, C1 to C6 alkylthio groups, amino groups, C1 to C6 alkylamino groups which may be mono- or di-substituted, arylamino groups which may be substituted, C4 to C9 cyclic amino groups which may have 1 to 3 hetero atoms, formylamino groups, C1 to C6 alkyl carbonylamino groups, C1 to C6 alkoxy carbonylamino groups, C1 to C6 alkylsulfonylamino groups and arylsulfonylamino groups which may be substituted.

The term "aromatic hydrocarbon group which may be substituted" used in this specification means an aromatic hydrocarbon group (there may be mentioned, for instance, a benzene ring, a naphthalene ring and an anthracene ring), which may have 1 to 5 substituents selected from the group consisting of halogen atoms, a hydroxyl group, a cyano group, a nitro group, C1 to C6 alkyl groups which may be substituted, C1 to C6 alkoxy groups, C1 to C6 alkylthio groups, and C2 to C6 dialkylamino groups.

The term "aromatic hetero ring which may be substituted" used in this specification means an aromatic hetero ring (there may be listed, for instance, 5- or 6-membered aromatic monocyclic hetero rings, which have 1 to 3 hetero atoms arbitrarily selected from the group consisting of nitrogen, oxygen and sulfur atoms, such as a pyridine ring, a pyrimidine ring, a pyridazine ring, a triazine ring, a quinoline ring, a naphthyridine ring, a quinazoline ring, an acridine ring, a pyrrole ring, a furan ring, a thiophene ring, an imidazole ring, a pyrazole ring, an oxazole ring, an isoxazole ring, a thiazole ring, an indole ring, a benzofuran ring, a benzothiazole ring, a benzimidazole ring, or a benzoxazole ring), which may have 1 to 5 substituents selected from the group consisting of halogen atoms, a hydroxyl group, a cyano group, a nitro group, C1 to C6 alkyl groups which may be substituted, C1 to C6 alkoxy groups, C1 to C6 alkylthio groups, and C2 to C6 dialkylamino groups.

The term "aliphatic hetero ring which may be substituted" used in this specification means an aliphatic hetero ring (there may be listed, for instance, 4 to 7-membered monocyclic aliphatic hetero rings or 9- or 10-membered aliphatic fused hetero rings which have 1 to 3 hetero atoms arbitrarily selected from the group consisting of nitrogen, oxygen and sulfur atoms, such as an azetidine ring, a pyrrolidine ring, a tetrahydrofuran ring, a piperidine ring, a morpholine ring, or a perazine ring), which may have 1 to 5 substituents selected from the group consisting of halogen atoms, a hydroxyl group, a cyano group, a nitro group, C1 to C6 alkyl groups which may be substituted, C1 to C6 alkoxy groups, C1 to C6 alkylthio groups, and C2 to C6 dialkylamino groups.

The term "C1 to C6 alkoxy group which may be substituted" used in this specification means a C1 to C6 alkoxy group which may have 1 to 5 substituents selected from the group consisting of halogen atoms, a hydroxyl group, a cyano group, C1 to C6 alkoxy groups, C1 to C6 alkylthio groups, amino groups, mono- or di-substituted C1 to C6 alkylamino groups, C4 to C9 cyclic amino groups, which may have 1 to 3 hetero atoms, formylamino groups, C1 to C6 alkyl carbonylamino groups, C1 to C6 alkylsulfonylamino groups and arylsulfonylamino groups which may be substituted.

As the foregoing "C1 to C6 alkoxy group," there may be listed a methoxy group, an ethoxy group, a butoxy group, a hexyloxy group and the like.

The term "aryloxy group which may be substituted" used in this specification means an aryloxy group which may have 1 to 5 substituents selected from the group consisting of halogen atoms, a hydroxyl group, a cyano group, a nitro group, C1 to C6 alkyl groups, C1 to C6 alkoxy groups and C1 to C6 alkylthio groups.

Examples of the foregoing "aryloxy group" include a phenoxy group, a naphthyloxy group and the like.

The term "arylamino group which may be substituted" used in this specification means an arylamino group which may have 1 to 5 substituents selected from the group consisting of halogen atoms, a hydroxyl group, a cyano group, a nitro group, C1 to C6 alkyl groups which may be substituted, C1 to C6 alkoxy groups and C1 to C6 alkylthio groups.

Examples of the foregoing "arylamino group" include a phenylamino group, a diphenylamino group, a biphenylamino group, a naphthylamine group and the like.

Examples of the foregoing "C1 to C6 alkylcarbonyl group" disclosed in this specification are a formyl group, an acetyl group, a propionyl group, a butyryl group, an isobutyryl group, a valeryl group and an isovaleryl group.

Examples of the foregoing "C1 to C6 alkoxycarbonyl group" disclosed in this specification are a methoxycarbonyl group, an ethoxycarbonyl group, a tert-butoxycarbonyl group and the like.

Examples of the foregoing "C1 to C6 alkylthio group" disclosed in this specification are a methylthio group, an ethylthio group, a propylthio group, an isopropylthio group and the like.

The term "mono- or di-substituted C1 to C6 alkylamino group" disclosed in this specification means a C1 to C6 alkylamino group which may have 1 to 2 substituents selected from the group consisting of halogen atoms, a hydroxyl group, a cyano group, C1 to C6 alkoxy groups, C1 to C6 alkylthio groups, amino groups, C1 to C6 alkylamino groups, C4 to C9 cyclic amino groups, which may have 1 to 3 hetero atoms, formylamino groups, C1 to C6 alkyl carbonylamino groups, C1 to C6 alkylsulfonylamino groups, arylsulfonylamino groups which may be substituted and the like.

Examples of the foregoing "C1 to C6 alkylamino group" used herein include a methylamino group, an ethylamino group, an n-propylamino group, an n-butylamino group, a sec-butylamino group, an n-pentyl-amino group, an n-hexylamino group and the like.

Examples of the foregoing "C1 to C6 alkylcarbonylamino group" disclosed in this specification are an acetylamino group, a propionylamino group, a butyryl amino group and the like.

Examples of the foregoing "C1 to C6 alkoxycarbonylamino group" disclosed in this specification are a methoxycarbonylamino group, an ethoxycarbonylamino group, a t-butoxycarbonylamino group, a hexyloxycarbonylamino group and the like.
Examples of the foregoing "C1 to C6 alkylsulfonylamino group" disclosed in this specification include a methylsulfonylamino group, an ethylsulfonylamino group and the like.

The term "arylsulfonylamino group which may be substituted" disclosed in this specification means an arylsulfonylamino group which may have 1 to 5 substituents selected from the group consisting of halogen atoms, a hydroxyl group, a cyano group, a nitro group, C1 to C6 alkyl groups, C1 to C6 alkoxy groups and C1 to C6 alkylthio groups.

Examples of the foregoing "arylsulfonylamino group" include a phenyl sulfonylamino group, a 4-methylphenylsulfonylamino group, a naphthyl sulfonylamino group and the like.

The term "C2 to C6 dialkylamino group" disclosed in this specification means a linear or branched dialkylamino group having 2 to 6 carbon atoms such as a dimethylamino group, a diethylamino group, a dipropylamino group, a diisopropylamino group and the like.

As the compound represented by the foregoing general formula (1), preferably used herein is (2S,4S)-1-[N-(4-ethoxycarbonylbicyclo[2.2.2]oct-1-yl)amino]acetyl-4-fluoropyridine-2-carbonitrile.

Examples of the foregoing "N-acylpyrrolidine carbonitrile derivative" disclosed in this specification other than those already described above include vildagliptin, saxagliptin, melogliptin, denagliptin, TS-021 and MP-513:

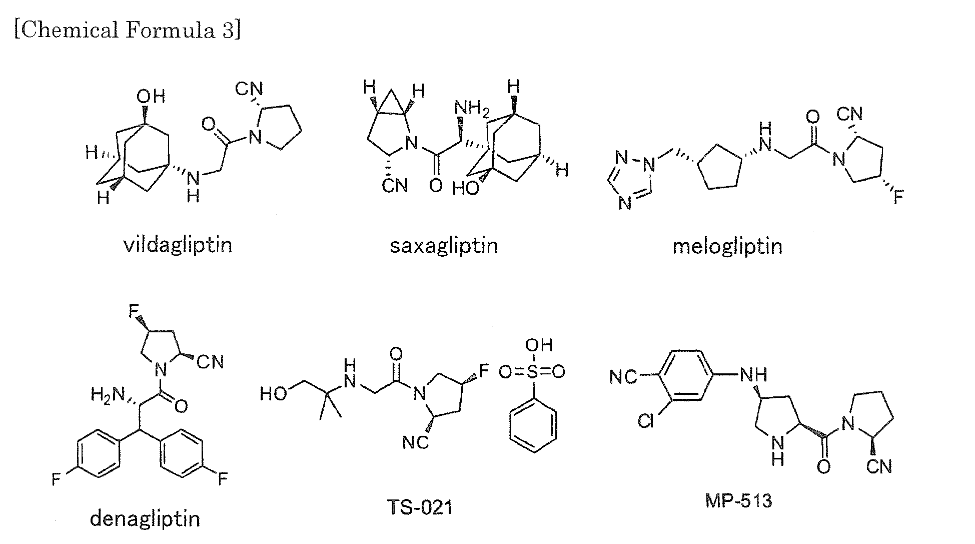

The term "basic normal salt hardly soluble in water" disclosed in this specification means a salt or a basic additive which is formed by a reaction of the hydrogen ion of an acid with the hydroxide ion of a base in proper quantities and which is hardly soluble in water. For instance, there may be listed calcium carbonate, magnesium carbonate, calcium citrate and the like, with calcium carbonate being preferably used herein. In this respect, it should be noted that these basic normal salts may be used alone or in any combination of at least two of them.

The term "hardly soluble in water" herein used means such a solubility that the amount of a solvent required for the complete dissolution of 1g or 1mL of a solute is not less than 1000mL. The term "solubility" herein used means the amount of the solute dissolved in the solvent within 30 minutes, while vigorously shaking the dispersion at a temperature of 20±5°C for 30 seconds at intervals of 5 minutes.

The amount of the basic normal salt to be incorporated into the matrix-type sustained release preparation according to the present invention is not restricted to any particular range, but it preferably ranges from 15 to 50% by mass, much preferably 15 to 30% by mass and further preferably 15 to 20% by mass relative to 100% by mass of the matrix-type sustained release preparation in order to retain the desired gel strength of the preparation and to eliminate any obstacle possibly encountered when compressing or tabletting the same.

The "excipient" disclosed in this specification includes crystalline cellulose, rice starch, potato starch, corn starch, wheat starch, α-starch, dextrin, dextran, sugar alcohols (such as mannitol, erythritol, xylitol, maltose, maltitol and sorbitol), glucose, fructose, maltose, lactose, isomerized lactose, reduced lactose, sucrose, talc, anhydrous silicic acid, anhydrous calcium phosphate, precipitated calcium carbonate, calcium silicate and the like. These excipients may be used alone or in any combination of at least two of them. As the foregoing excipients, preferably used herein include lactose or sugar alcohols (such as mannitol, erythritol, xylitol, maltose, maltitol and sorbitol), with lactose or mannitol being much preferably used herein. In addition, sugar alcohols are preferred from the viewpoint of the stabilization of the compound, with mannitol being further preferred.

The term "hydrogeling agent" disclosed in this specification means a polymer having an ability to get swollen through the absorption of water and is composed of a polymer material which, when brought into contact with water or other aqueous solvents, can undergo swelling to some extent through the absorption of such water/solvent. Examples of such hydrogeling agents include Hypromellose, hydroxy propyl cellulose, hydroxyethyl cellulose, methyl cellulose, sodium carboxymethyl cellulose, polyvinyl pyrrolidone, polyethylene glycol, polyvinyl alcohol, xanthane gum, guar gum, carrageenan, polyethylene oxides, carboxy vinyl polymers, or their analogues and the like. Preferred are pH-independent hydrogeling agents such as Hypromellose from the viewpoint of the ability thereof to reduce the pH-dependent release. These hydrogeling agents may be used alone or in any combination of at least two of them.

The amount of the hydrogeling agent to be incorporated into the matrix-type sustained release preparation of the present invention is not limited to, but preferably not more than 3 times the amount of the basic normal salt used, from such a point of view that the pH-dependent release of the resulting matrix-type sustained release preparation is reduced, and the hydrogeling agent is much preferably used in an amount of not more than 2 times the amount of the basic normal salt used.

In the matrix-type sustained release preparation according to the present invention, it is also possible to optionally incorporate, into the same, other substances such as a binder, a lubricant, a film-coating material, a sugar coat-applying material, a plasticizer, a coloring agent, a flavoring agent, an odor-modifying agent or an essence, in so far as they do not impair the desired effect of the present invention.

Examples of such binders usable in the present invention include hydroxypropyl cellulose, Hypromellose, povidone, polyvinyl pyrrolidone, methyl cellulose, polyvinyl alcohol, carboxymethyl cellulose, partially pre-gelatinized starch, pre-gelatinized starch, sodium alginate, pullulan, powdered gum Arabic, gelatin, dextrin and the like.
Examples of forgoing lubricants usable in the present invention include magnesium stearate, calcium stearate, sucrose esters of fatty acids and the like.

Examples of the foregoing film-coating materials which may be used in the present invention include methyl cellulose, ethyl cellulose, hydroxypropyl cellulose, Hypromellose and polyvinyl pyrrolidone.
Examples of the foregoing sugar coat-applying materials include sucrose, trehalose, lactose, mannitol, powdered hydrogenated maltose starch syrup and the like.

In the present invention, when the matrix-type sustained release preparation is subjected to a film-coating or sugar-coating operation, it is also possible to, if necessary, incorporate, into the preparation, an excipient, a plasticizer, a coloring agent and the like. Such excipients include, for instance, talc, calcium carbonate, titanium oxide and the like.
Examples of such plasticizers are Macrogol 6000, Copolyvidone, triethyl citrate and the like.
Examples of such coloring agents are titanium oxide, Food Yellow No. 5, Food Blue No. 2, iron sesquioxide, yellow iron sesquioxide and the like.
Examples of the foregoing flavoring agents, which can be used in the present invention, include white soft sugar, sorbitol, xylitol, citric acid, ascorbic acid, tartaric acid, malic acid, aspartame, acesulfame potassium, thaumatin, saccharin sodium, dipotassium glycyrrhizinate, sodium glutamate, sodium 5'-inosinate, disodium 5'-guanylate and the like.

Examples of the foregoing odor-modifying agents usable in the present invention are trehalose, malic acid, maltose, potassium gluconate, anise essence, vanilla essence, cardamon essence and the like.
Examples of the foregoing essences usable in the present invention are lemon oil, orange oil, mentha oil, menthol and the like.

### Examples:

Now, the present invention will hereunder be described in more detail with reference to the following Examples and Comparative Examples, but the scope of the present invention is not limited by the Examples to and Comparative Examples at all. In the following Examples, (2S,4S)-1-[N-(4-ethoxy-carbonyl bicyclo[2.2.2]oct-1-yl)amino]acetyl-4-fluoropyridine-2-carbonitrile can be prepared according to the method as disclosed in WO 2005/075421, Pamphlet.

### Example 1

According to the formulations specified in the following Table 1, there were blended, using a mortar, (2S,4S)-1-[N-(4-ethoxycarbonylbicyclo [2.2.2]oct-1-yl)amino]acetyl-4-fluoropyridine-2-carbonitrile (hereunder simply referred to as "Compound 1"), Hypromellose (available from Shin-Etsu Chemical Co., Ltd. under the trade names of Metolose 60SH-50 and Metolose 60SH-4000), mannitol (available from ROQUETTE Company under the trade name of Pearitol 200SD), calcium carbonate (available from KANTO Chemical Co., Ltd.) for a period of 3 minutes, followed by, in order, the addition of stearyl alcohol (available from Nippon Oil and Fats Co., Ltd. under the trade name of NAA-45) which had been milled using a crushing type granulating and spherizing machine (available from OKADA SEIKO Industrial Co., Ltd. under the trade name of ND-10 or ND-30S) using screen sizes of 500 µm and 250 µm and magnesium stearate (available from TAIHEI Chemical Industial Co., Ltd. under the trade name of Magnesium Stearate Vegetable), and the subsequent blending of these ingredients for one minute. Furthermore, these ingredients were further blended in a polyethylene bag for 30 seconds and then the resulting blend was compressed into tablets each having a weight of 300mg using a tablet machine (available from HATA Iron works Co., Ltd. under the trade name of HT-AP-18-SSII) equipped with R-face punches of 12 mm radius of curvature and 9 mm in diameter under the lower punch compression force of 1100 kg.

### Example 2

According to the formulations specified in the following Table 1, the same procedures used in Example 1 were carried out except that lactose hydrate (available from Fonterra. Ltd. under the trade name of LACTOSE 200M) was substituted for the mannitol and that the pressure upon the compression was set at a level ranging from 980 to 1050kg to thus form a desired tablet.

### Example 3

According to the formulations specified in the following Table 3, there were blended, using a mortar, Compound 1, Hypromellose (available from Shin-Etsu Chemical Co., Ltd. under the trade names of Metolose 60SH-50 and Metolose 60SH-4000), lactose hydrate (available from Fonterra. Ltd. under the trade name of LACTOSE 200M), and calcium carbonate (available from KANTO Chemical Co., Ltd.) for a period of 3 minutes, followed by, in order, the addition of stearyl alcohol (available from Nippon Oil and Fats Co., Ltd. under the trade name of NAA-45) which had been milled using a crushing type granulating and spherizing machine (available from OKADA SEIKO Industrial Co., Ltd. under the trade name of ND- 10 or ND-30S) using screen sizes of 500 µm and 250 µm and magnesium stearate (available from TAIHEI Chemical Industrial Co., Ltd. under the trade name of Magnesium Stearate Vegetable) and the subsequent blending of these ingredients for one minute. Furthermore, these ingredients were further blended in a polyethylene bag for 30 seconds and then the resulting blend was compressed into tablets each having a weight of 250mg using a tablet machine (available from HATA Iron works Co., Ltd. under the trade name of HT-AP-18-SSII) equipped with R-face punches of 10 mm radius of curvature and 8 mm in diameter under the lower punch compression force of 1100 kg.

### Example 4

According to the formulations specified in the following Table 3, the same procedures used in Example 3 were repeated except that the weight of the resulting tablet was set at a level of 280mg to thus form a desired tablet.

### Comparative Example 1

According to the formulations specified in the following Table 1, there were blended, using a mortar, Compound 1 and Hypromellose (available from Shin-Etsu Chemical Co., Ltd. under the trade names of Metolose 60SH-50 and Metolose 60SH-4000), for a period of 3 minutes, followed by, in order, the addition of stearyl alcohol (available from Nippon Oil and Fats Co., Ltd. under the trade name of NAA-45) which had been milled using a crushing type granulating and spherizing machine (available from OKADA SEIKO Industrial Co., Ltd. under the trade name of ND-10 or ND-30S) using screen sizes of 500 µm and 250 µm and magnesium stearate (available from TAIHEI Chemical Industrial Co., Ltd. under the trade name of Magnesium Stearate Vegetable) and the subsequent blending of these ingredients for one minute. Furthermore, these ingredients were further blended in a polyethylene bag for 30 seconds and then the resulting blend was compressed into tablets each having a weight of 183mg using a tablet machine (available from DATA Iron works Co., Ltd. under the trade name of HT-AP-18-SSII) equipped with R-face punches of 12 mm radius of curvature and 9 mm in diameter under the lower punch compression force ranging from 900 to 1020kg.

### Comparative Example 2

According to the formulations specified in the following Table 1, there were blended, using a mortar, Compound 1, Hypromellose (available from Shin-Etsu Chemical Co., Ltd. under the trade names of Metolose 60SH-50 and Metolose 60SH-4000) and mannitol (available from ROQUETTE Company under the trade name of Pearitol 200SD), for a period of 3 minutes, followed by, in order, the addition of stearyl alcohol (available from Nippon Oil and Fats Co., Ltd. under the trade name of NAA-45) which had been milled using a crushing type granulating and spherizing machine (available from OKADA SEIKO Industrial Co., Ltd. under the trade name of ND-10 or ND-30S) using screen sizes of 500 µm and 250 µm and magnesium stearate (available from TAIHEI Chemical Industrial Co., Ltd. under the trade name of Magnesium Stearate Vegetable) and the subsequent blending of these ingredients for one minute. Furthermore, these ingredients were further blended in a polyethylene bag for 30 seconds and then the resulting blend was compressed into tablets each having a weight of 300mg using a tablet machine (available from HATA Ironworks Co., Ltd. under the trade name of HT-AP-18-SSII) equipped with R-face punches of 12 mm radius of curvature and 9 mm in diameter under the lower punch compression force of 1100 kg.

### Comparative Example 3

According to the formulations specified in the following Table 1, the same procedures used in Example 1 were carried out except that lactose hydrate (available from Fonterra. Ltd. under the trade name of LACTOSE 200M) was substituted for the mannitol and the blend powders were compressed using the same punches under the compression force of 1100kg to obtain tablets.

### Comparative Example 4

According to the formulations specified in the following Table 2, the same procedures used in Example 1 were carried out except that sodium hydrogen carbonate (available from KANTO Chemical Co., Ltd.) was substituted for calcium carbonate to thus give tablets.

### Comparative Example 5

According to the formulations specified in the following Table 2, the same procedures used in Example 1 were carried out except that trisodium citrate di-hydrate (available from KANTO Chemical Co., Ltd.) was substituted for calcium carbonate to thus give tablets.

### Comparative Example 6

According to the formulations specified in the following Table 2, the same procedures used in Example 1 were carried out except that disodium hydrogen phosphate (available from KANTO Chemical Co., Ltd.) was substituted for calcium carbonate to thus give tablets.

### Comparative Example 7

According to the formulations specified in the following Table 2, the same procedures used in Example 1 were carried out except that anhydrous calcium hydrogen phosphate (available from Kyowa Chemical Industry Co., Ltd.) was substituted for the calcium carbonate to thus give tablets.

### Comparative Example 8

According to the formulations specified in the following Table 3, the same procedures used in Example 1 were carried out except that magnesium oxide (available from KANTO Chemical Co., Ltd.) was substituted for calcium carbonate to thus give tablets.

### Comparative Example 9

According to the formulations specified in the following Table 3, the same procedures used in Example 1 were carried out except that sodium sulfate (available from KANTO Chemical Co., Ltd.) was substituted for the calcium carbonate to thus give tablets.

### Table 1: Formulation 1

| Component | Comp. Ex.1 | Comp. Ex.2 | Comp. Ex.3 | Ex. 1 | Ex. 2 |
|---|---|---|---|---|---|
| Compound 1 | 30 | 30 | 30 | 30 | 30 |
| Hypromellose (Metolose 60SH-50) | 37.5 | 37.5 | 37.5 | 37.5 | 37.5 |
| Hypromellose (Metolose 60SH -4000) | 112.5 | 112.5 | 112.5 | 112.5 | 112.5 |
| Mannitol | -- | 117 | -- | 67 -- | |
| Lactose hydrate | -- | -- | 117 | -- | 67 |
| Calcium carbonate | -- | -- | -- | 50 | 50 |
| Stearyl alcohol | 1.2 | 1.2 | 1.2 | 1.2 | 1.2 |
| Magnesium stearate | 1.8 | 1.8 | 1.8 | 1.8 | 1.8 |
| Total (mg) | 183 | 300 | 300 | 300 | 300 |

### Table 2: Formulation 2

| Component | Comparative Example | | | |
|---|---|---|---|---|
| | 4 | 5 | 6 | 7 |
| Compound 1 | 30 | 30 | 30 | 30 |
| Hypromellose (Metolose 60S H-50) | 37.5 | 37.5 | 37.5 | 37.5 |
| Hypromellose (Metolose 60S H-4000) | 112.5 | 112.5 | 112.5 | 112.5 |
| Mannitol | 42 | 42 | 42 | 42 |
| Sodium hydrogen carbonate | 75 | -- | -- | -- |
| Trisodium citrate dihydrate | -- | 75 | -- | -- |
| Disodium hydrogen phosphate | -- | -- | 75 | -- |
| Calcium hydrogen phosphate | -- | -- | -- | 75 |
| Stearyl alcohol | 1.2 | 1.2 | 1.2 | 1.2 |
| Magnesium stearate | 1.8 | 1.8 | 1.8 | 1.8 |
| Total (mg) | 300 | 300 | 300 | 300 |

### Table 3: Formulation 3

| Component | Comparative Ex. | | Example | |
|---|---|---|---|---|
| | 8 | 9 | 3 | 4 |
| Compound 1 | 30 | 30 | 30 | 30 |
| Hypromellose (Metolose 60SH-50) | 37.5 | 37.5 | 25 | 25 |
| Hypromellose (Metolose 60SH-4000) | 112.5 | 112.5 | 75 | 75 |
| Mannitol | 42 | 42 | -- | -- |
| Lactose hydrate | -- | -- | 67 | 97 |
| Magnesium oxide | 75 | -- | -- | -- |
| Sodium sulfate | -- | 75 | -- | -- |
| Calcium carbonate | -- | -- | 50 | 50 |
| Stearyl alcohol | 1.2 | 1.2 | 1.2 | 1.2 |
| Magnesium stearate | 1.8 | 1.8 | 1.8 | 1.8 |
| Total (mg) | 300 | 300 | 250 | 280 |

### Test Example 1: Stability Test

The stability tests were carried out according to the following method. The results thus obtained are summarized in the following Table 4.

### Test Method

Tablets were put in a glass bottle and then they were stored for 4 weeks in an environment maintained under conditions of 40°C and 75% RH while the bottle was tightly sealed or it was left open. Subsequently, the amount of the decomposition products originated from Compound 1 was determined according to the liquid chromatography technique. The content thereof was expressed in terms of the percentage relative to the content of Compound 1. The tablets were extracted with a diluted phosphoric acid (1 → 1000)/acetonitrile-mixed liquid (7:3) for liquid chromatography. In this respect, there was a detection limit on the order of 0.05% in the content of the decomposition products, and therefore, no account was taken for the decomposition product of less than the detection limit in the determination of the content thereof.

### Test Conditions for Liquid Chromatography

Column Used: Inertsil ODS-3V, having an inner diameter of 4.6mm and a length of 150mm, and a particle size of 5 µm; available from GL Science Corporation;
Mobile Phase, Liquid A: A diluted solution (27→12500) of sodium 1-octane sulfonate in a phosphoric acid solution (1→1000);
Mobile Phase, Liquid B: Acetonitrile for liquid chromatography;
Supply of Mobile Phase: A concentration gradient was controlled during the term extending from the injection of the sample to 60 minutes after the injection, while changing the amounts of the liquid A from 80% by volume to 60% by volume and the liquid B from 20% by volume to 40% by volume, respectively.
Detector: An ultraviolet-visible absorption spectrometer (detection wavelength: 210nm).

### Table 4: Results of Stability Test Carried out after Storage at 40°C/75% RH for 4 weeks

| Item | Content (%) of Decomposition Products observed under Open Conditions | Content (%) of Decomposition Products observed under Sealed Conditions |
|---|---|---|
| Ex. 1 | Not Detected | Not Detected |
| Ex. 2 | Not Detected | Not Detected |
| Ex. 3 | No Data | No Data |
| Ex. 4 | Not Detected | Not Detected |
| Comp. Ex. 1 | Not Detected | Not Detected |
| Comp. Ex. 2 | Not Detected | Not Detected |
| Comp. Ex. 3 | Not Detected | Not Detected |
| Comp. Ex. 4 | 0.11 | Not Detected |
| Comp. Ex. 5 | Not Detected | Not Detected |
| Comp. Ex. 6 | 0.05 | 0.06 |
| Comp. Ex. 7 | Not Detected | Not Detected |
| Comp. Ex. 8 | 0.19 | 0.12 |
| Comp. Ex. 9 | Not Detected | Not Detected |

### Test Example 2: Dissolution Test

The dissolution test was carried out according to the method detailed below. The results thus obtained are summarized in the following Tables 5 and 6.

### Test Method:

The dissolution test was carried out according to the paddle method at a rotational frequency of 100 revolutions per minute, while using, as test liquids, 900mL each of a first liquid for the dissolution test and a second liquid for the dissolution test. In this respect, the tablets as samples were put in a sinker for use in the dissolution test. After 1, 2, 3, 4, 5 and 6 hours from the initiation of the dissolution test, each test sample was collected through a membrane filter having a pore size of 0.45 µm and the dissolution rate was determined according to the liquid chromatography technique under the following test conditions:

### Test Conditions for Liquid Chromatography

Column Used: Inertsil ODS-3V, having an inner diameter of 4.6mm and a length of 150mm, and a particle size of 5 µm; available from GL Science Corporation;
Mobile Phase, Liquid A: A diluted solution (27→12500) of sodium 1-octane sulfonate in a phosphoric acid solution (1→1000);
Mobile Phase, Liquid B: Acetonitrile for liquid chromatography;
Supply of Mobile Phase: The mixing ratio of the liquid A and the liquid B was maintained (Liquid A: Liquid B = 7:3).
Detector: An ultraviolet-visible absorption spectrometer (detection wavelength: 210nm).

**Table 5: Results Obtained in Examples**

| Ex. No. | Liquid Used for Dissolution Test | Time (Hr) | | | | | |
|---|---|---|---|---|---|---|---|
| | | 1 | 2 | 3 | 4 | 5 | 6 |
| 1 | First liquid | 21 | 33 | 44 | 53 | 61 | 68 |
| | Second liquid | 10 | 17 | 24 | 30 | 36 | 41 |
| | Difference | 11 | 16 | 20 | 23 | 25 | 27 |
| 2 | First liquid | 19 | 31 | 41 | 49 | 56 | 62 |
| | Second liquid | 10 | 17 | 23 | 28 | 33 | 38 |
| | Difference | 9 | 14 | 18 | 21 | 23 | 24 |
| 3 | First liquid | 20 | 32 | 42 | 50 | 58 | 65 |
| | Second liquid | 11 | 19 | 26 | 33 | 39 | 44 |
| | Difference | 9 | 13 | 16 | 17 | 19 | 21 |
| 4 | First liquid | 23 | 35 | 45 | 54 | 62 | 69 |
| | Second liquid | 15 | 24 | 32 | 39 | 45 | 51 |
| | Difference | 8 | 11 | 13 | 15 | 17 | 18 |

| | | | | | | | |
|---|---|---|---|---|---|---|---|
| Unit of numerical values: %. | | | | | | | |

**Table 6: Results Obtained in Comparative Examples**

| Compo. Ex. No. | Liquid Used for Dissolution Test | Time (Hr) | | | | | |
|---|---|---|---|---|---|---|---|
| | | 1 | 2 | 3 | 4 | 5 | 6 |
| 1 | First liquid | 30 | 45 | 56 | 66 | 73 | 79 |
| | Second liquid | 8 | 14 | 20 | 25 | 31 | 36 |
| | Difference | 22 | 31 | 36 | 41 | 42 | 43 |
| 2 | First liquid | 32 | 48 | 60 | 70 | 78 | 83 |
| | Second liquid | 11 | 19 | 27 | 34 | 41 | 48 |
| | Difference | 21 | 29 | 33 | 36 | 37 | 35 |
| 3 | First liquid | 29 | 43 | 55 | 64 | 72 | 78 |
| | Second liquid | 10 | 18 | 25 | 33 | 40 | 47 |
| | Difference | 19 | 25 | 30 | 31 | 32 | 31 |
| 4 | First liquid | 17 | 29 | 38 | 46 | 54 | 61 |
| | Second liquid | 10 | 17 | 25 | 32 | 40 | 47 |
| | Difference | 7 | 12 | 13 | 14 | 14 | 14 |
| 5 | First liquid | 27 | 41 | 53 | 62 | 70 | 76 |
| | Second liquid | 10 | 18 | 26 | 34 | 41 | 48 |
| | Difference | 17 | 23 | 27 | 28 | 29 | 28 |
| 6 | First liquid | 25 | 39 | 51 | 62 | 70 | 77 |
| | Second liquid | 18 | 26 | 35 | 45 | 54 | 63 |
| | Difference | 7 | 13 | 16 | 17 | 16 | 14 |
| 7 | First liquid | 27 | 41 | 52 | 61 | 68 | 74 |
| | Second liquid | 10 | 16 | 22 | 28 | 33 | 38 |
| | Difference | 17 | 25 | 30 | 33 | 35 | 36 |
| 8 | First liquid | 16 | 24 | 32 | 39 | 45 | 52 |
| | Second liquid | 7 | 12 | 18 | 23 | 28 | 32 |
| | Difference | 9 | 12 | 14 | 16 | 17 | 20 |
| 9 | First liquid | 29 | 43 | 54 | 63 | 71 | 77 |
| | Second liquid | 13 | 22 | 30 | 38 | 45 | 51 |
| | Difference | 16 | 21 | 24 | 25 | 26 | 26 |

| | | | | | | | |
|---|---|---|---|---|---|---|---|
| Unit of numerical values: %. | | | | | | | |

The foregoing results indicate that, in case where calcium carbonate was used (Examples 1 to 4), the resultant preparations are stable, without generating decomposition products (Table 4) and that the difference in dissolution rate between the first and second liquids is not more than 16% during the term of 0 to 2 hours and the pH-dependent release of the sample is reduced (Table 5).

On the other hand, the results indicate that, in case where no basic additive was used (Comparative Examples 1 to 3); where trisodium citrate dihydrate was used (Comparative Example 5); where calcium hydrogen phosphate was added (Comparative Example 7); and where sodium sulfate was added (Comparative Example 9), the samples were stable without generating decomposition products (Table 4), but the drug release properties from samples were pH-dependent and the difference in dissolution rate between the first and second liquids was high (Table 6). Moreover, in case where sodium hydrogen-carbonate was used (Comparative Example 4), where disodium hydrogen citrate was used (Comparative Example 6); and where magnesium oxide was used (Comparative Example 8), the pH-dependent release could be reduced (Table 6), but decomposition products were formed and accordingly the stabilization effect was insufficient (Table 4).

### Test Example 3: Test for Determining Gel Strength

The gel strength was calculated according to the method disclosed in HARM TECH JAPAN Vol.20, No.13 (2004), pp. 135-140. More specifically, the gel strength was determined according to the method comprising the steps of immersing and allowing, to stand, a tablet in 100mL of a first liquid (the first liquid specified in the 15^{th} revised edition of Japanese Pharmacopoeia, pH 1.2) or the second liquid (the second liquid specified in the 15^{th} revised edition of Japanese Pharmacopoeia, pH 6.8), while maintaining the temperature thereof at 40°C in a thermostatic chamber for time periods of 4, 8 and 16 hours; The immersed tablets (hydrogelled tablets) were crushed using a parallel plate viscometer (spreadmeter), and then the diameter of the crashed tablets was measured. The gel strength of samples was determined by dividing the diameter of initial tablet by the diameter of immersed tablet. The results thus determined are summarized in the following Table 7.

**Table 7: Gel Strength**

| Kind of Liquid | First Liquid | | | Second Liquid | | |
|---|---|---|---|---|---|---|
| Immersion Time (Hr) | 4 | 8 | 16 | 4 | 8 | 16 |
| Example 1 | 0.4 | 0.2 | 0.2 | 0.5 | 0.2 | 0.2 |
| Example 4 | 0.4 | ND | ND | 0.3 | 0.2 | 0.2 |

| | | | | | | |
|---|---|---|---|---|---|---|
| ND: Cannot be determined. | | | | | | |

The data as shown in Table 7 clearly indicate that the tablets prepared in Examples 1 and 4 still have sufficient gel strength even after the immersion in the first liquid for 4 hours or after the immersion in the second liquid for 8 hours.

## Claims

1. A matrix-type sustained release preparation wherein an N-acylpyrrolidine carbonitrile derivative having an amino group in a molecule is stabilized, said preparation comprising a basic normal salt hardly soluble in water and an excipient.

2. The matrix-type sustained release preparation of claim 1, wherein said N-acylpyrrolidine carbonitrile derivative having an amino group in a molecule is vildagliptin, saxagliptin, melogliptin, denagliptin, TS-021, MP-513, or an aminoacetyl-pyrrolidine carboxamide derivative represented by the following general formula (1): 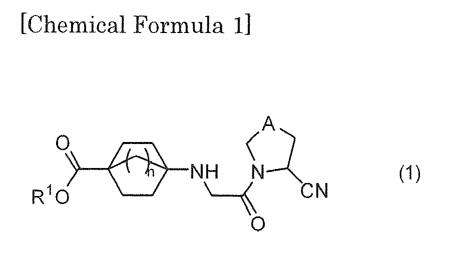 (In the general formula (1),
A represents CH₂, CHF or CF₂;
R¹ represents a hydrogen atom, a C1 to C6 alkyl group which may be substituted, a C3 to C8 cycloalkyl group which may be substituted, an arylmethyl group which may be substituted, an arylethyl group which may be substituted, an aromatic hydrocarbon group which may be substituted, an aromatic hetero ring which may be substituted, or an aliphatic hetero ring which may be substituted; and n is 1 or 2).

3. The matrix-type sustained release preparation of claim 1, wherein said N-acylpyrrolidine carbonitrile derivative having an amino group in a molecule is vildagliptin, saxagliptin, melogliptin, denagliptin, TS-021, MP-513, or (2S,4S)-1-[N-(4-ethoxycarbonylbicyclo[2.2.2]oct-1-yl)amino]acetyl-4-fluoro pyridine-2-carbonitrile.

4. The matrix-type sustained release preparation of any one of claims 1 to 3, wherein said basic normal salt hardly soluble in water is calcium carbonate.

5. The matrix-type sustained release preparation of any one of claims 1 to 4, wherein said excipient is lactose or a sugar alcohol.

6. The matrix-type sustained release preparation of any one of claims 1 to 5, wherein said excipient is lactose or mannitol.

7. The matrix-type sustained release preparation of any one of claims 1 to 6, further comprising a pH-independent hydrogeling agent.

8. The matrix-type sustained release preparation of any one of claims 1 to 7, wherein the amount of said hydrogeling agent to be incorporated is not more than 3 times that of said basic normal salt hardly soluble in water.

9. The matrix-type sustained release preparation of claim 8, wherein the amount of said hydrogeling agent to be incorporated is not more than 2 times that of said basic normal salt hardly soluble in water.
